(19) Europäisches Patentamt
European Patent Office
Office européen des brevets

(11) Publication number: 0 352 766
A2

(12) EUROPEAN PATENT APPLICATION

(21) Application number: 89113785.3

(22) Date of filing: 26.07.89

(51) Int. Cl.4: C07H 15/10 , G01N 33/53 , G01N 33/531

(30) Priority: 26.07.88 IT 4823188

(43) Date of publication of application: 31.01.90 Bulletin 90/05

(84) Designated Contracting States: AT BE CH DE ES FR GB GR IT LI LU NL SE

(71) Applicant: FIDIA S.p.A.
Via Ponte della Fabbrica 3-A
I-35031 Abano Terme (Padova)(IT)

(72) Inventor: Della Valle, Francesco
Via Cerato 14
Padova(IT)
Inventor: Kirschner, Gunter
Via Leonardo da Vinci 14
35031 Abano Terme Padova(IT)

(74) Representative: Vossius & Partner
Siebertstrasse 4 P.O. Box 86 07 67
D-8000 München 86(DE)

(54) **Synthesis of glycosphingolipid derivates for use in the preparation of immuno- and affinity adsorbents to be used in the purification of antibodies and specific toxins, and for diagnostic use.**

(57) A lycosphingolipid derivative is disclosed which is useful in binding glycosphingolipids to an immunoadsorbent matrix. The process for preparing the immunoadsorbent leaves the oligosaccharide and lipid components of the glycosphingolipid unaltered after conjugation with the solid support matrix, while at the same time permitting a covalent and stable bond between the glycolipid and solid matrix.

EP 0 352 766 A2

# SYNTHESIS OF GLYCOSPHINGOLIPID DERIVATES FOR USE IN THE PREPARATION OF IMMUNO- AND AFFINITY ADSORBENTS TO BE USED IN THE PURIFICATION OF ANTIBODIES AND SPECIFIC TOXINS, AND FOR DIAGNOSTIC USE

Glycosphingolipids are normal components of all animal cells and some vegetable cells. Their distribution on the cell membrane enables them to regulate the interactions between cells and the environment.

Glycosphingolipids are made up of an oligosaccharide portion and a lipid portion, joined by a glycoside bond. The oligosaccharide portion is made up of one or more neutral carbohydrates to which an acidic saccharide or another functional group may be bound. The lipid or ceramide portion is made up of a long-chain aminoalcohol (sphingosine) and by a long-chain fatty acid, joined by an amide bond; it is highly hydrophobic as a result of the presence of two long hydrocarbyl chains.

The glycosphingolipids which contain a single monosaccharide unit are defined by names which identify their sugar content, for example galactosylceramide and glucosylceramide; similarly the glycosphingolipid which contains lactose is known as lactosylceramide. More complex glycosphingolipids are identified by the oligosaccharide common to one particular family of compounds, using a prefix to identify the family and a suffix to identify the number of glycoside units in the chain.

Table 1 shows the structures of the main families with their complete saccharide sequences, including the order of their single sugars and the position and anomeric configuration of their glycoside bonds.

These basic structures also pertain to those glycosphingolipids having more than four or less than four sugars.

Table 1

| Prefix | Abbreviation | Structure |
|---|---|---|
| lacto | Lc | Gal(β1→3)GlcNAc(β1→3)Gal(β1→4)Glc |
| lactoneo | Lcn | Gal(β1→4)GlcNAc(β1→3)Gal(β1→4)Glc |
| muco | Mc | Gal(β1→3)Gal(β1→4)Gal(β1→4)Glc |
| gala | Ga | GalNAc( 1→3)Gal( 1→4)Gal(α1→4)Gal* |
| globo | Gb | GalNAc(β1→3)Gal(α1→4)Gal(β1→4)Glc |
| globoiso | Gbi | GalNAc(β1→3)Gal(α1→3)Gal(β1→4)Glc |
| ganglio | Gg | Gal(β1→3)GalNAc(β1→4)Gal(β1→4)Glc |

Glycosphingolipids can also be subdivided into neutral glycosphingolipids, gangliosides (that is, glycosphingolipids containing one or more sialic acid residues) and sulfatoglycosphingolipids

Recently acquired knowledge of the antigenic and receptoral properties of glycosphingolipids (Rapport M. M. and Graf L., Immunochemical reactions of lipids. Prog. Allergy, 13, 273-331, 1969; Marcus D.M. and Schwarting G.A., Immunochemical properties of glycolipids and phospholipids, Adv. Immunol. 23, 203-240, 1976), and particularly the involvement of gangliosides in the processes of specific recognition of signal molecules on the membrane surface and the transduction of signals through the membrane, have aroused considerable interest in the study of these compounds (Tetamanti G., Sonnino R., Ghidoni R., Masserini M. and Venerando B., In: V. Degiorgio and M. Corti (Eds), physics of Amphiphiles: Micelles, Vesicles and Microemulsions, North--Holland, Amsterdam, 1985, pp. 607-636).

Purified antibodies directed against a single glycosphingolipid are important means for studying localization of specific glycosphingolipids on the cell membrane. They are used also in the determination and quantification of glycosphingolipids for clinical uses in patients and could be a basis for specific immunological therapy.

It is also known that many toxins specifically bind to particular glycosphingolipids such as gangliosides. Examples of the various toxins include the following: cholera toxin, tetanus toxin, botulinic toxin and the toxin derived from Escherichia coli.

For the purification of antiglycosphingolipid antibodies, and in particular of antiganglioside antibodies, and for the preparation of highly purified toxins, various methods are currently being used having in common the use of said immunoadsorbents or affinity adsorbents in which the glycosphingolipid component is bound in a stable manner. A first method is described by D.M. Marcus in Application of

immunological techniques to the study of glycosphingolipids. In: Glycolipid Methodology, L. A. Witting Ed., Am. Oil Chem. Soc., Champaign, Ill., pp. 223-245 (1976) and uses the incorporation of glycolipids in a polyacrylamide gel. A second method uses a covalent bond between the carboxy group of an acidic glycolipid and aminoalkyl-agarose (Parikh Z. and Cuatrecases P., Ganglioside-agarose and cholera toxin. In; Methods of Enzymology. W.B. Jacoby and M. Wilcheck Eds. Academic press, New York 34, 610-619 (1974) and Cuatrecases and Parikh, Affinity chromotagraphy of Vibrio Cholerae enterotoxin-ganglioside polysaccharide and the biological effects of ganglioside-containing soluble polymers, U.S.P. 4, 125, 492; 14 Nov. 1978) or a silica gel functionalized with an aminopropylic group (S.K. Kundu and S.K. Roy, Aminopropylsilica gel as a solid support for preparation of glycolipid immunoadsorbent and purification of antibodies, J. Lipid Res. 20, 825-833, 1979). A third method (Laine R. A., G. Yogeeswaran and S. Hakomori, Glycosphingolipids covalently linked to agarose gel or glass beads, J. Biol. Chem. 249, 4460-4466, 1974), uses a system by which the double olefinic bond of the sphingosine is oxidized and the resulting "acidic glycolipid" is conjugated with an aminic group of a solid support.

A fourth method (Tayot J. L., Tardy M., Isolation of cholera toxin by affinity chromatography on porous silica beads with covalently coupled ganglioside $GM_1$. Adv. Exp. Med. Biol. 125, 471 (1980) and Tayot J. L., Tardy M. in Material sur reversiblen Fixierung biologischer Makromolekule, Verfahren su seiner Herstellung und seine Verwendung German Patent No. 28 40 503 dated 29.03.1979), uses the formation of de-N-acetylated and de-N-acylated products (lyso compounds) to bring about a reaction between the aminic group which is thus released and a reactive group of the matrix.

A fifth method (Taki T., Abe M., Matsumoto M., Simple purification method for anti-glycolipid antibody using polystyrene latex beads coated with gangliotetrasylceramide. J. Biochem. 91, 1813 (1982) and Hirabayashi et al. A new method for purification of anti-glycosphingolipid antibody. Avian anti-hematoside (neu Gc) antibody, J. Biochem. 94, 327 (1983) uses a hydrophobic bond between the glycosphingolipid and polyester or octyl-Sepharose-4B lactics.

All these immobilization methods, despite their overall validity, present various drawbacks. Marcus's method and that of Taki and Hirabayashi use the formation of a physical bond between the glycosphingolipid and the solid matrix. Consequently, although the chemical structure of the lipid itself remains unaltered, its stability is short-lived.

The methods of Cuatrecasas, Kundu, Hakomori and Tayot on the other hand, allow a stable bond to be formed between the glycosphingolipid and the matrix, but in doing this they profoundly alter either the oligosaccharide component (Cuatrecasas and Kundu) or the lipid component (Hakamori) or both (Tayot).

An ideal immunoadsorbent must leave unaltered both the oligosaccharide and the lipid components after conjugation with the solid support, at the same time permitting a covalent and therefore stable bond between glycolipid and solid matrix.

It is an object of the present invention to provide a derivation method which satisfies this primary and fundamental requirement and overcomes the above disadvantages of prior methods.

The present invention is directed to a process of preparing glycosphingolipid derivatives, and in particular ganglioside derivatives useful in the preparation of immuno and affinity adsorbents.

The process comprises:

(a) N-deacylating and/or N-deacetylating a glycosphingolipid to form a glycosphingolipid lysoderivative; and

(b) reacylating said glycosphingolipid lysoderivative with a $C_4$-$C_{22}$ carboxylic acid containing a functional group to form said glycosphingolipid derivative.

The invention is also directed to a process of preparing a glycosphingolipid immuno and affinity adsorbent which comprises:

(a) preparing a glycosphingolipid lysoderivative, that is, a derivative obtained by removal of the N-acyl and N-acetyl groups of the glycosphingolipid, or in other words a deacyl-deacetyl-derivative;

(b) reacylating a free amino group of the long chain base moiety of the glycosphingolipid lysoderivative with a long-chain carboxylic acid containing a protected functional group;

(c) reacylating the remaining free amino group of the carbohydrate moiety;

(d) deprotecting the functional group; and

(e) conjugating the reacylated lysoderivative with a suitable immunoadsorbent matrix.

The invention is also directed to a glycosphingolipid immuno and affinity adsorbent which includes a glycosphingolipid lysoderivative bound to an adsorbent matrix. The glycospingolipid immuno and affinity adsorbent is prepared by the above described process.

The invention is also directed to a method of purifying and isolating an antiglycosphingolipid antibody which comprises:

(a) running an antiglycosphingolipid antibody through the above prepared glycosphingolipid immuno

and affinity adsorbent; and

(b) purifying said antiglycosphingolipid antibody.

Examples of the antiglycosphingolipid antibodies to be used in the present method are antiganglioside antibodies. Particularly preferred is the antiglycosphingolipid antibody anti-GM.

The invention is also directed to a method of purifying a toxin which specifically binds to glycosphingolipids which comprises:

(a) running a toxin which specifically binds to glycospingolipids through a glycosphingolipid immuno and affinity adsorbent according to claim 22; and

(b) purifying said toxin.

Examples of various toxins useful in the present method are described herein above.

Examples of the neutral glycosphingolipids include the following: 1-0-glycosylceramides, 1-0-diglycosylceramides, 1-0-triglycosylceramides, 1-0-tetraglycosylceramides, 1-0-pentaglycosylceramides, and the fucosides which include Lewis antigens and the substances of blood groups H, A and B.

Examples of the gangliosides, include the following: galactosylgangliosides, lactosylgangliosides, the gangliosides containing N-acetylgalactosamine (ganglio series), the gangliosides containing N-acetylglucosamine (lacto series), fucogangliosides and sulfatogangliosides.

Examples of the sulfatoglycosphingolipids include the following: 1-0-galactosylceramide sulfate, 1-0-triglycosylceramide sulfate.

Examples of useful eluant solvents systems useful for liberating either or both of the antiglycosphingolipid antibody and toxin are citrate buffer, chaotropic agents such as quanidine, and area, or any other releasing agents which can split the hydrophobic bond between the toxin and the antibody.

Fig. 1 is an electrophoretic analysis of the total retainment of cholera toxin and complete purification after elation with citrate buffer.

According to preferred embodiments of the invention:

(1) The glycosphingolipid can be any one of glycosphingolipids which are compounds per se known, such as those described above. preferred glycosphingolipids are the ganglosides. Particularly preferred in the present invention is the ganglioside $GM_1$

The glycosphingolipid lysoderivative can be prepared by a process which will remove the N-acyl and N-acetyl groups on the glycosphingolipid. For instance, it is known that gangliosides have an N-acyl group and other N-acetyl groups and that these groups can be removed, for example, by hydrolysis, converting the groups into $NH_2$ groups. In particular, the N-acyl and N-acetyl groups can be removed by alkaline hydrolysis as described in Holmgren, Mansson, and Svennerholrh, Medical Biology (1974) 52, 229-233.

(2) Reacylation of the lysoderivative is accomplished by the use of a long-chain carboxylic acid, effectively causing condensation of the lysoderivative with the acid. Long-chain carboxylic acids particularly useful in the invention are those having a functional group in addition to the carboxyl group, especially those having the additional functional group terminally placed with respect to the carboxyl group. Examples of such functional groups are an amine group, a hydroxyl, a sulfhydryl, and an anhydride. Useful long-chain carboxylic acids may include fatty acids, i.e., those carboxylic acids have an alkyl chain of from 4 to 22 carbon atoms with a terminal-COOH group, such as butyric ($C_4$), lauric ($C_{12}$), palmitic ($C_{16}$) and stearic ($C_{18}$), whereby the lysoderivative would be prepared by use of the corresponding fatty acid amine.

The reacylation reaction is particularly accomplished by reaction of the glycosphingolipid lysoderivative with an activated and protected long chain carboxylic acid, that is a carboxylic acid in which the carboxyl group is activated and the amine group is protected. The aminic group can be protected with known protecting groups, for example protecting groups having a carbonyl halide or carbonyl-chloride group capable of forming an amide bonded protecting group on said amine group, such as 9-fluorenylmethylchloroformate

The carboxyl group can be activated with known activating groups, for example groups having a group reactive with a carboxyl group, such as di-N-succinimidyl carbonate.

(3) Conjugation of the glycosphingolipid to the matrix is accomplished by suitable reaction of a glycosphingolipid derivative with the matrix in active form.

Preferably an activated matrix is reacted with the glycosphingolipid acylated with the carboxylic acid as discussed above. In this manner, the activated matrix containing a reactive group can, for example, react with the additional, preferably terminal, functional group such as an amine in the carboxylic acid group.

The activated matrix is preferably activated Sepharose, but may also be any activated polysaccharide polymer, or a modified polysaccharide polymer such as dextran, starch or cellulose. The activated matrix may particularly have the general formula

$$R^1\text{-O-}\overset{\overset{\displaystyle O}{\|}}{C}\text{-}R^2\text{-}Ⓟ$$

wherein $R^1$ represents a carboxyl protecting group, $R^2$ represents an acyl or alkyl group and Ⓟ represents

the matrix support. $R^2$ can particularly represent an acyl or alkyl having a terminal $-NH_2$ group for reacting with and bonding to the matrix. A particular feature of the activated matrix is that it is activated so as to be reactive with the reacylated glycosphingolipid, that is, reactive with the glycosphingolipid having been acylated with the long-chain carboxylic acid.

The process of the invention can be represented by the following general scheme.

glycosphingolipid(I) → glycosphingolipid lysoderivative (II)

+ long-chain carboxylic acid (activated and protected derivative thereof (III))

↓

activated matrix

reacylated glycosphingolipid (IV) condensed with long-chain carboxylic acid

↓

glycosphingolipid-matrix conjugate

Considering $GM_1$ as the representative glycosphingolipid and aminolauric acid as the representative long-chain carboxylic acid, the process of the invention can be represented by the following scheme.

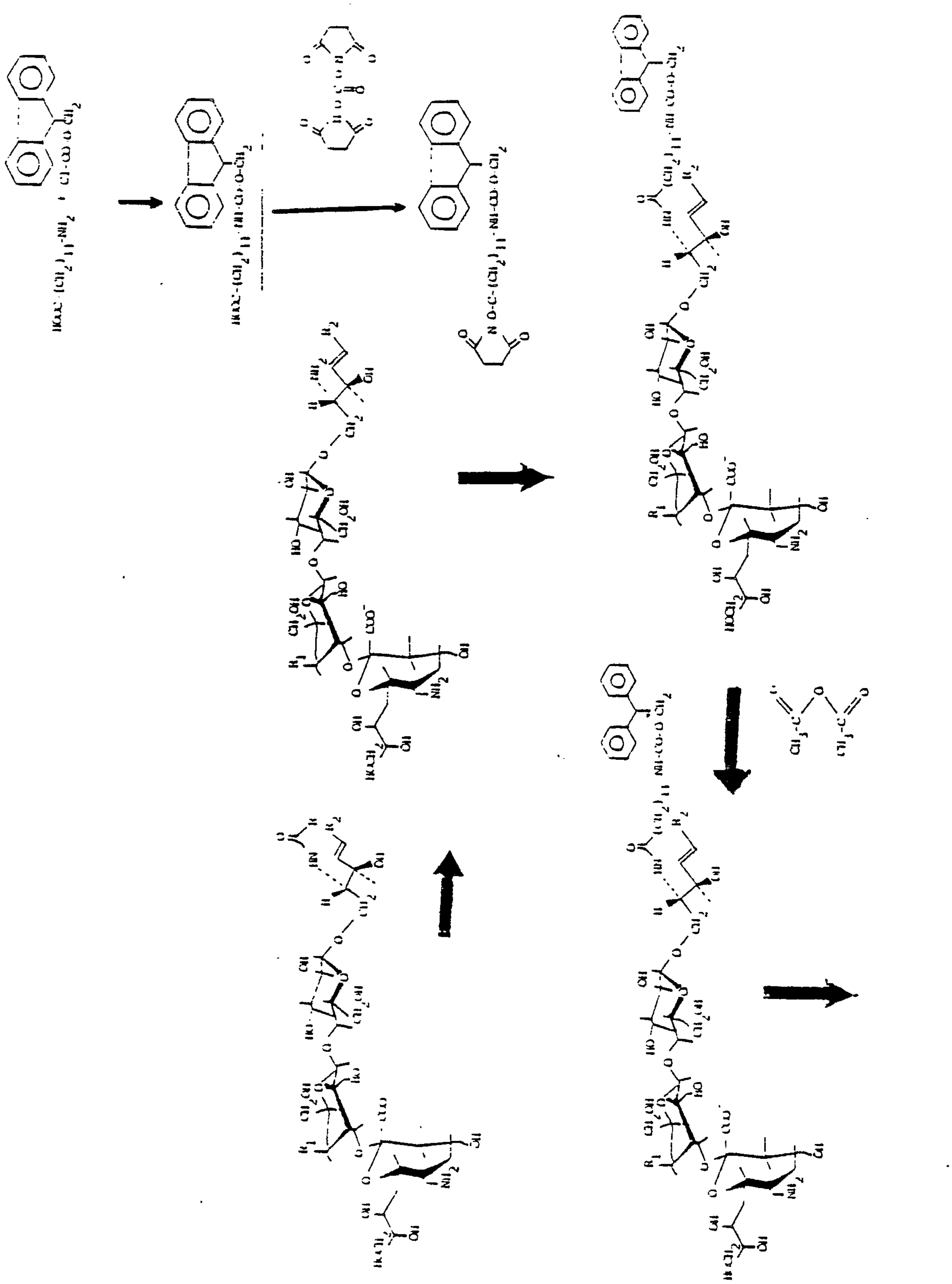

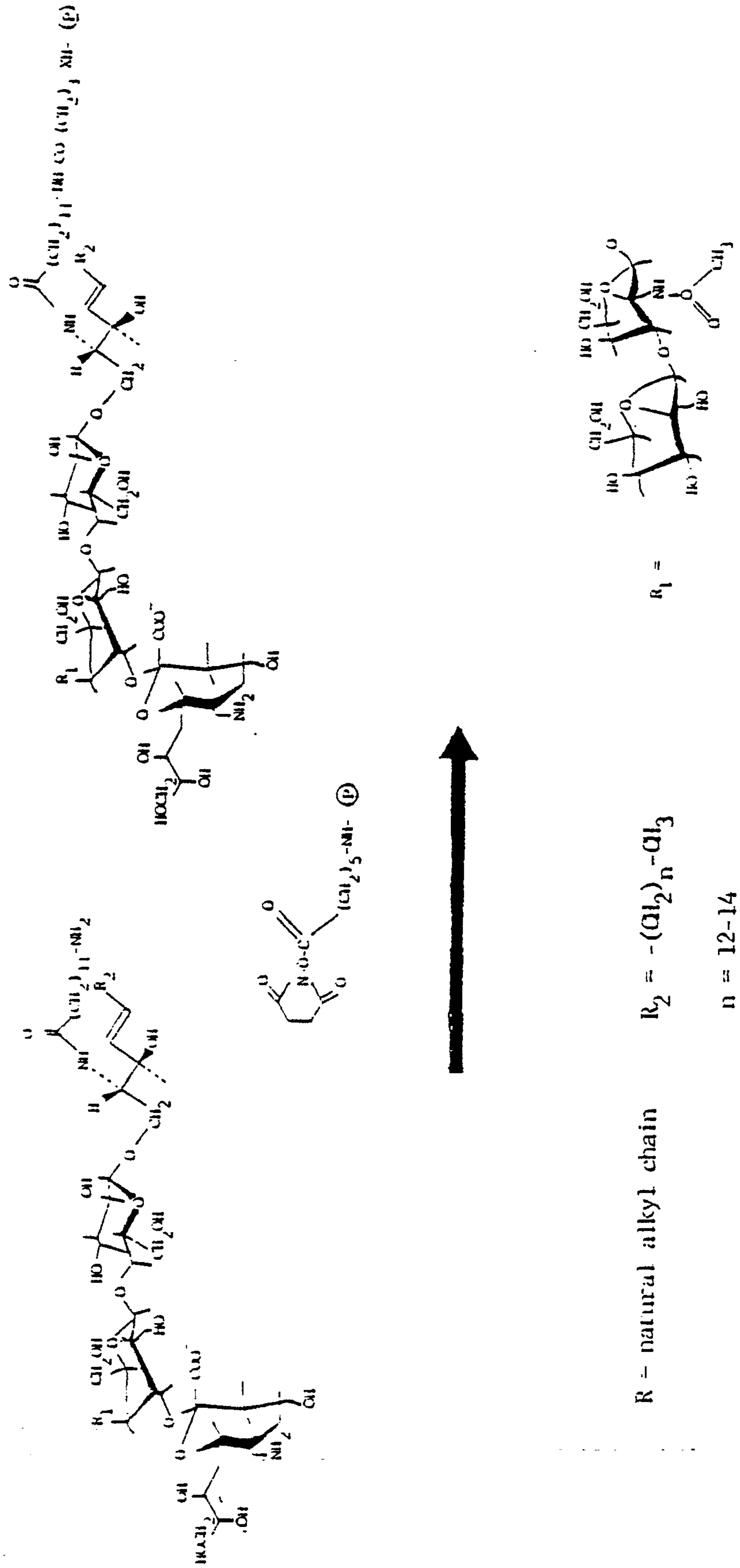

R = natural alkyl chain

$R_2 = -(CH_2)_n-CH_3$

$n = 12-14$

The following examples describe the preparation of: an activated and protected fatty acid (Example 1); the lysoderivative of a ganglioside; the subsequent condensation of lysoderivatives with an activated fatty acid (Examples 2-4); the final conjugation of the glycosphingolipids thus modified with a solid matrix (Examples 5-7); the purification of antiglycosphingolipid antibodies (in particular anti-$GM_1$) (Example 8) and the purification of a toxin (in particular the cholera toxin) (Example 9) by means of these new immunoadsorbents or affinity adsorbents.

EXAMPLE 1

Preparation of aminolauric acid (ALA) activated on the carboxy group and protected on the aminic group.

(a) Preparation of the fluorenylmethoxycarbonyl derivative of aminolauric acid (ALA-Fmoc)

2 gr (9.3 mmol) of aminolauric acid are dispersed in 150 ml of $NaHCO_3$ 1% and placed in ice under constant agitation. Once dispersion has become homogenous, 2.6 g (11.2 mmol) of 9-fluorenylmethylcholorformate dissolved in 350 ml of tetrahydrofurane (THF) are slowly added. Dispersion is maintained at room temperature under constant agitation.

Any undissolved material is discarded by filtration and the solution is partitioned with 250 ml of ethyl ether; the aqueous phase is washed with 250 ml of ethyl ether and the two organic phases are gathered together. The organic phase is vacuum dried and redissolved in 5ml of THF. The product is then precipitated by adding 100 ml of HC1 (1 N). The white precipatate is washed with water and recrystallized by hexane/toluene 80:25. 2.15 g of ALA-Fmoc are obtained, corresponding to a yield of 54%.

(b) Preparation of the succinimidyl derivative of ALA-Fmoc

1 g (2.3 mmol) of ALA-Fmoc are solubilized in 150 ml THF and to the resulting solution are added 0.4 ml (4.6 mmol) of triethylamine and 0.7 g (2.7 mmol) of di-N-succinimidylcarbonate in 100 ml of acetone.

The reaction mixture is kept at room temperature under agitation for 18 hours and then vacuum dried. The succinimidyl derivative of ALA-Fmoc is recrystallized by ethyl acetate. 1.15 g of product are obtained, corresponding to a yield of 85%.

EXAMPLE 2

Preparation of galactosylceramide (ALA)

200 mg (434 µmol) of 1-O-galactosylsphyngosine (pyschosin) are dissolved in 3 ml of anhydrous dimethylformamide and then mixed with 20 g of Triton X 100, 1.007 g (1.9 mmol) of succinimidyl derivative of ALA-Fmoc dissolved in 15 ml of anhydrous tetrahydrofurane and 85 ul triethylamine.

The mixture is made to react under constant agitation for 24 hours at room temperature. The solution is concentrated at 2 ml in a vacuum and then precipitated in 50 ml of ethyl acetate. The product is chromatographed on silica gel 100 column using chloroform/methanol/water 110:40:6 as elution solvent.

The fractions containing the pure product are gathered, vacuum-dried, taken up again with 2 ml of chloroform/methanol (1:1) and precipitated in 50 ml of ethyl acetate. 174 mg are obtained, corresponding to a yield of 45%.

The product obtained is dissolved in 10 ml of chloroform/methanol (1:1) and anhydrous vapours of ammonia are bubbled through the solution for 2 hours. The reaction mixture if maintained at room temperature for another 18 hours. The solution is concentrated to about 1 ml in vacuum and the product, galactosylceramide (ALA), is precipitated in 10 ml of acetone, Quantitatiave yield.

Gas-chromatographical analysis confirms the chemical composition of the product, showing the presence of galactose, sphingosine and aminolauric acid in a molar ratio of 1:1:1.

EXAMPLE 3

Preparation of ganglioside $GM_1$(ALA)

200 mg (150 µmol) of deacetyl-deacyl-$GM_1$ dissolved in 3 ml of anhydrous DMF are mixed with 2.0 g of Triton X 100, 350 mg (660 umol) of succinimidyl derivative of ALA-Fmoc dissolved in 5 ml of anhydrous tetrahydrofurane and 30 ul of triethylamine. The deacetyl-$GM_1$ (ALA-Fmoc) thus obtained is precipated in cold ethyl acetate.

100 mg of deacetyl-$GM_1$ (AlLA-Fmoc) are dissolved in 10 ml of chloroform/methanol (1:1) and 15 ul of acetic anhydride. The reaction mixture is kept under constant agitation for 3 hours at room temperature and then almost completely vacuum dried at a temperature of 37°C. The $GM_1$ (ALA-Fmoc) is precipitated by adding acetone. Quantitative yield.

100 mg of $GM_1$ (ALA-Fmoc) are dissolved in 5 ml of chloroform/methanol 1:1 and anhydrous ammonia vapours are bubbled through the solution for 2 hours. The reaction mixture is maintained at room temperature for another 18 hours. The solution is concentrated in vacuum to about 1 ml and the product $GM_1$ (ALA) is precipitated in 10 ml of acetone. Quantitative yield.

Gas-chromatographic analysis confirms the chemical position of the product, showing the presence of glucose, galactose, N-acetylgalactosamine, N-acetylneuraminic acid, sphingosine and aminolauric acid in a ratio of 1:2:1:1:1:1.

EXAMPLE 4

Preparation of galactosylceramide $1^3$-sulfate (ALA)

200 mg (365 umol) of 1-O-galactosylsphingosine-$I^3$-sulphate (lyso-sulphatide) dissolved in 3 ml of anhydrous DMF are mixed with 2.0 g of Triton X 100, 832 mg (1.57 mmol) of succinimidyl derivative of ALA-fmoc dissolved in 5 ml of anhydrous tetrahydrofurane and 70 ul of triethylamine.

The mixture is reacted under constant agitation for 24 hours at room temperature. The solution is concentrated in vacuum to a volume of 2 ml and then precipitated in 50 ml of ethyl acetate. The product is chromatographed on silica gel 100 column using chloroform/methanol/water (110:40:6) as a solvent.

The fractions containing the pure product are gathered, vacuum dried, taken up with 2 ml of chloroform/methanol 1:1 and anhydrous vapours of ammonia bubbled through the solution for 2 hours. The reaction mixture is kept at room temperature for another 18 hours. The solution is concentrated to about 1 ml in a vacuum and the product, galactosylceramide-$I^3$-sulphate (ALA), is precipitated in 10 ml of acetone. Quantitative yield.

Gas-chromatographic analysis confirms the chemical composition of the product, showing the presence of galactose, sphingosine and aminolauric acid in a molar ratio of 1:1:1. The presence of the sulphate group is determined by ionic chromatography.

EXAMPLE 5

Preparation of an immunoadsorbent conjugated with galactosylceramide (ALA)

100 mg of galactosylceramide (ALA) are conjugated with 5 g of activated CH-Sepharose 4B (Pharmacia, Uppsala, Sweden) according to the following procedure: 5 g of activated CH-Sepharose are suspended in HC1 $10^{-3}$M and the additives present are washed away on filtre using about 100 ml of HC1 $10^{-3}$M. The binding galactosylceramide (ALA) is dissolved in 100 ml of $NaHCO_3$ 0.2 M, NaCl 1 M/tetrahydrofurane 1:4 and mixed with the gel; it is kept under agitation for 24 hours at room temperature. The gel is then washed with buffer and the residue active groups are fixed with ethanolamine (1 M, pH 9). The product is washed with three cycles of alternating pH. Each cycle consists in washing with pH 4 (acetate buffer 0.1 M containing NaCl 1 M) followed by a washed with pH 8.0 (Tris 0.1 M buffer containing NaC1 1 M).

EXAMPLE 6

Preparation of an immunoadsorbent conjugated with $GM_1$ (ALA)

100 mg of $GM_1$ (ALA) are conjugated with 5 g of activated CH-Sepharose 4B (Pharmacia, Uppsala, Sweden) according to the following procedure: 5 g of activated CH-Sepharose 4B are suspended in HC1 $10^{-3}$M and the additives present are washed away on a filtre using about 1000 ml of HC1 $10^{-3}$M. The binding $GM_1$ (ALA) is dissolved in 100 ml of $NaHCO_3$ 0.2 M, NaC1 (1 M)/methanol (1:1) and mixed with the gel. It is kept under agitation for 24 hours at room temperature. The gel is then washed with buffer and the residue active groups fixed with ethanolamine (1 M, pH 9). The product is washed with 3 cycles of alternating pH. Each cycle consists of one wash with pH 4 (acetate buffer 0.1 M containing NaC1 1 M) followed by a wash with pH 8.0 (Tris 0.1 M buffer containing NaCl 1 M).

EXAMPLE 7

Preparation of an immunoadsorbent conjugated with galactosylceramide-$I^3$-sulphate (ALA)

100 mg of galactosylceramide-$I^3$-sulphate (ALA) are conjugated with 5 g of activated CH-Sepharose 4B (Pharmacia, Uppsala, Sweden) according to the following procedure: 5 g of activated CH-Sepharose 4B are suspended in HC1 $10^{-3}$M and the additives present are washed away on a filtre using about 1000 ml of HC1 $10^{-3}$M. The binding galactosylceramide-$I^3$-sulphate (ALA) is dissolved in 100 ml of $NaHCO_3$ 0.2 M, NaCl 1 M/tetrahydrofurane 1:4 and mixed with the gel; it is kept under agitation for 24 hours at room temperature. The gel is then washed with buffer and the residue active groups fixed with ethanolamine (1 M, pH 9). The product is washed with 3 cycles of alternating pH. Each cycle consists of one wash with pH 4 (acetate buffer 0.1 M containing NaCl 1 M) followed by a wash with pH 8.0 (Tris 0.1 M buffer containing NaCl 1M).

EXAMPLE 8

Preparation of anti-$GM_1$ antibodies and their preparative purification

-- Immunogenic preparation

The method consists of the following:

Phosphatidylcholine from eggs, cholesterol and $GM_1$, solubilized in chloroform/methanol (2:1, v.v.), are mixed and then vacuum dried at a temperature of 37°C. The lipid film is kept in high vacuum overnight in order to remove all traces of solvent. A phosphate buffered solution 50 mM, pH 7.0, of methylated bovine serum albumin, 1 mg/ml is added. The final molar ratio between phosphatidylcholine, cholesterol, $GM_1$ and MBSA is 1:4:0.1:0.02. The lipid film is suspended by means of an vortex agitator and the solution is sonicated in nitrogen for 20 minutes, until it is clear.

Six rabbits, hybrid HY/CR (Charles River, Calco, Italy) are injected intravenously into the marginal ear vein with 1 ml of the immunogenic preparation, 3 times a week for 4 weeks. Samples of blood are drawn during the period of immunization to follow the progress of the antibody titer. Blood samples are then drawn from the rabbits by cardiac puncture five days after receiving the 12th injection. The serum thus obtained is heated to 65°C for two hours.

The activity of these original sera and of the subsequent fractions purified by affinity chromatography is tested both by passive agglutination and by the ELISA technique.

A mixture of glycolipid antigen, phosphatidylcholine and cholesterol in a molar ratio of 1:12:0.27 is used for the passive agglutination test; agglutination is determined by Leitz microscope magnified 30 times.

The ELISA method used was a modification of Holmgren and Svennerholm' s procedure (1973): polyester plates for microtitration are coated with 0.2 ug of glycolipid antigen in ethanol, left to evaporate, washed with PBS-Tween 20 and then saturated with 1% polyvinylpyrrolidone.

A series of dilutions of the original sera or of the purified antibodies are added to the single plates and incubated overnight at 4°C. After thorough washing, a secondary antirabbit antibody obtained from goat and conjugated with peroxidase is added (Coppel Lab. Cochranville, pA) diluted 1:200 and incubated at 4°C for 2 hours. O-phenylenediamine is used as colorimetric substrate; 15 minutes later the reaction is stopped with $H_2SO_4$ 4 N and the optical density read at 490 nm.

EXAMPLE 9

-- Affinity purification

1 ml of $GM_1$ affinity chromatographic resin, loaded into a glass syringe which had been previously washed with guanidine-HC1 and NaCl 0.5 M in acetic acid 1N, is balanced with Tris buffer (Tris 0.1 M + NaCl 0.1 M + EDTA 0.01 M brought to pH 7.3 with HC1).

3.5 ml of anti $GM_1$ antiserum from rabbit, diluted with an equal volume of Tris buffer, are repeatedly loaded onto a column at a temperature of 4°C. After washing with Tris buffer until the D.O. is less than 0.010, specific elution is affected by 0.5 M NaCl in 1 M acetic acid.

The solution thus obtained is dialized against PBS and concentrated by ultrafiltration.

Recovery of anti $GM_1$ antibody activity and the proteic content after affinity purification, are reported in Table 1.

The data refer to 2 different anti $GM_1$ antisera, tested by the agglutination technique and ELISA; in the last column is reported the degree of purification in terms of antibody titer in respect to the protein concentration.

The titers against other glycolipids which have a cross reaction with $GM_1$ in the original sera, and more precisely $GD_{Ib}$ and asialo $GM_1$, were also analyzed: Table 2 shows the ratios between anti $GM_1$ activity and that of anti $GD_{Ib}$ and anti asialo $GM_1$.

TABLE II

| | | Anti-GM1 Titer Anti-GDIb Titer | | Anti-GM1 Titer Anti-AsialoGM1 Titer | |
|---|---|---|---|---|---|
| | | Before Affinity Chrom. | After | Before | After |
| S.1 | AGGL. | 1 | 2.0 | 1 | 2.0 |
| | ELISA | 0.5 | 2.0 | 0.5 | 2.0 |
| S.2 | AGGL. | 2.0 | 4.0 | 2.0 | 4.0 |
| | ELISA | 1 | 2.0 | 1 | 4.0 |

Part of the unrecovered activity is found in the fraction not withheld by the column (Table 3).

Regarding cross reactivity, after purification there is a two- to four-fold increase in the titer against $GM_1$ compared to the titer against $GD_{Ib}$ and asialo $GM_1$.

TABLE III

| | Recovery of Antibody Activity Against $GM_1$ in % | | % of Activity in the Break Through of The Affinity Chromat. | | % of Protein Recovery | % Purif. (Titer Over Protein) | |
|---|---|---|---|---|---|---|---|
| | AGGL. | ELISA | AGGL. | ELISA | | AGGL. | ELISA |
| S.1 | 50% | 36% | 21% | 7% | 0.6% | 83 | 60 |
| S.2 | 80% | 70% | 12.5% | 4% | 0.56% | 143 | 125 |

EXAMPLE 10

Purification of the cholera toxin by immunoadsorbent conjugated with $GM_1$.

Syncase fermentation medium and the Inaba 569B Vibrio Cholerae strain were used. After fermentation and treatment with merthiolate, the medium is centrifuged and filtered on Millipore membrane. It is then concentrated to about a tenth of its original volume on Amicon PM 10 Membrane.

25 ml of concentrate are then passed over a chromatographic column containing 2 ml of $GM_1$-Sepharose affinity resin, previously washed with Tris/HC1 buffer 20 mM pH 7.5.

After loading (1 ml/min flow) the column is washed with Tris/HC1 20 mM pH 7.5 and then the bound toxin is eluted with citrate buffer 100 mM pH 2.8.

Electrophoretic analysis shows total retainment of the toxin by the immunoadsorbent and its complete purification after elution with citrate buffer, as seen from line 3 in Fig. 1.

The high specificity of the immunoadsorbents here described, which is the object of the present invention, is due to the exact chemical characterization of the reaction intermediates, to a derivation reaction which does not alter the chemical structure of the oligosaccharide or the lipid component, to the theoretical yield of the immobilization reaction and to the stability in time and during repeated use of the final product.

The preparation method specifically described herein for obtaining immunoadsorbents or affinity adsorbents having as active agent a particular neutral glycosphingolipid such as galactosylceramide (cerebroside), a particular glycosphingolipid containing sialic acid (ganglioside $GM_1$), or a particular sulphated glycosphingolipid such as sulphatide, can be extended to the other glycosphingolipids belonging to the same classes of which the above-mentioned derivatives are representative examples.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. A process for preparing a glycosphingolipid derivative which comprises:

(a) N-deacylating and/or N-deacetylating a glycosphingolipid to form a glycosphingolipid lysoderivative; and

(b) reacylating said glycosphingolipid lysoderivative with a $C_4$-$C_{22}$ carboxylic acid containing a functional group to form said glycosphingolipid derivative.

2. The process according to claim 1, wherein said functional group is an amino group located at the terminal position of the lipid chain.

3. The glycosphingolipid derivative prepared by the process of claim 1 or 2.

4. A process for preparing a glycosphingolipid immuno and affinity adsorbent which comprises:

(a) N-deacylating and/or N-deacetylating a glycosphingolipid to form a glycosphingolipid lysoderivative;

(b) reacylating a free amino group of the long chain base moiety of the glycosphingolipid lysoderivative with a long-chain carboxylic acid containing a protected functional group;

(c) reacylating the remaining free amino group of the carbohydrate moiety;

(d) deprotecting the functional group; and

(e) conjugating the reacylated lysoderivative with a suitable immunoadsorbent matrix.

5. The process according to claim 4, wherein said glycosphingolipid is a ganglioside.

6. The process according to claim 5, wherein said ganglioside is $GM_1$.

7. The process according to claim 1, wherein said functional group in said long chain carboxylic acid is an amino group.

8. The process according to claim 1, wherein said functional group of said long chain acid is terminal with respect to said carboxylic acid group.

9. The process according to any one of claims 4 to 6, wherein said long chain carboxylic acid which is protected at said functional group is activated at said carboxylic acid group.

10. The process according to claim 7, wherein said long chain carboxylic acid is activated at said carboxylic group and protected at said amine group.

11. The process according to any one of claims 4 to 6 or 9, wherein said long chain carboxylic acid having a functional group is aminolauric acid.

12. A glycosphingolipid immuno and affinity adsorbent which comprises:

(a) an adsorbent matrix, and

(b) a glycosphingolipid derivative bound to said adsorbent matrix.

13. The glycosphingolipid immuno and affinity adsorbent according to claim 12, wherein said glycosphingolipid is a ganglioside.

14. The adsorbent according to claim 13, wherein said ganglioside is $GM_1$.

15. Use of the immunoadsorbent as in any one of claims 12 to 14 for the purification of anti-glycosphingolipid antibodies.

16, Use of the immunoadsorbent as in any one of claims 12 to 14 for the purification of anti-ganglioside antibodies.

17, Use of the affinity adsorbent as in any one of claims 12 to 14 for the purification of toxins which bind specifically to glycosphingolipids.

18. Use of an affinity adsorbent as in claim 12 in which the conjugated glycosphingolipid is the ganglioside $GM_1$, for the purification of the cholera toxin.

19. Use of an immunoadsorbent as in any one of claims 12 to 14 for the qualitative and quantitative determination of anti-glycosphingolipid antibodies.

20. Use of an immunoadsorbent as in claim 13 for the qualitative and quantitative determination of anti-ganglioside antibodies.

28.08.89

Neu eingereicht / Newly f
Nouvellement déposé
(R 35)

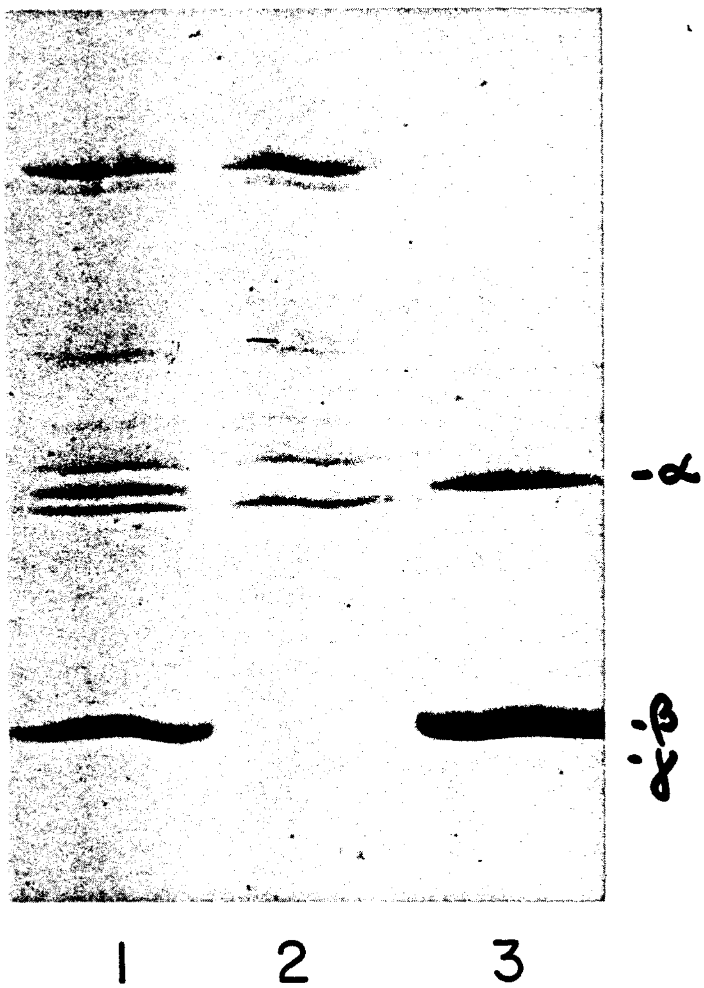


FIG.1